# EUROPEAN PATENT SPECIFICATION

(11) **EP 1 833 502 B1**
(45) Date of publication and mention of the grant of the patent: **02.03.2011**
(21) Application number: 05850944.9
(22) Date of filing: 22.12.2005
(51) Int. Cl.: A61K 38/17, A61P 11/00

(54) **PHARMACEUTICAL COMPOSITION FOR THE TREATMENT OF INVASIVE PULMONARY ASPERGILLOSIS**
PHARMAZEUTISCHE ZUSAMMENSETZUNG ZUR BEHANDLUNG VON INVASIVER LUNGENASPERGILLOSE
COMPOSITION PHARMACEUTIQUE POUR LE TRAITEMENT DE L'ASPERGILLOSE PULMONAIRE INVASIVE

(30) Priority: 23.12.2004 IN DE25342004
(43) Date of publication of application: 19.09.2007
(73) Proprietor: Council of Scientific and Industrial Research, Special Institutional Area New Delhi 110067 (IN)
(72) Inventor: SARMA, Puranam Usha, Delhi 110007 (IN); GUPTA, Taruna Madan, Delhi 110007 (IN); KAUR, Savneet, Delhi 110007 (IN); THIEL, Steffen, DK-411008 Aarhus (DK)
(74) Representative: Kinkeldey, Daniela
(86) International application number: PCT/IN2005/000429
(87) International publication number: WO 2006/067807

(56) References cited:
- WO-A-00/69894
- WO-A-03/033522
- WO-A-03/090774
- WO-A-2004/089394
- US-A- 5 270 199
- US-A1- 2002 086 817
- US-A1- 2004 029 785
- VALDIMARSSON H ET AL: "RECONSTITUTION OF OPSONIZING ACTIVITY BY INFUSION OF MANNAN-BINDINGLECTIN (MBL) TO MBL-DEFICIENT HUMANS" SCANDINAVIAN JOURNAL OF IMMUNOLOGY, BLACKWELL SCIENCE PUBL., OXFORD, GB, vol. 48, no. 2, August 1998 (1998-08), pages 116-123, XP000911946 ISSN: 0300-9475
- CROSDALE DANIEL J ET AL: "Mannose-binding lectin gene polymorphisms as a susceptibility factor for chronic necrotizing pulmonary aspergillosis" JOURNAL OF INFECTIOUS DISEASES, vol. 184, no. 5, 1 September 2001 (2001-09-01), pages 653-656, XP009062991 ISSN: 0022-1899
- NETH O ET AL: "Interaction of human mannose-binding lectin (MBL) to organisms isolated from immunocompromised children" MOLECULAR IMMUNOLOGY, vol. 35, no. 6-7, April 1998 (1998-04), page 362, XP009062992 & XVII INTERNATIONAL COMPLEMENT WORKSHOP; RHODES, GREECE; OCTOBER 11-16, 1998 ISSN: 0161-5890
- NETH O ET AL: "MANNOSE-BINDING LECTIN BINDS TO A RANGE OF CLINICALLY RELEVANT MICROORGANISMS AND PROMOTES COMPLEMENT DEPOSITION" INFECTION AND IMMUNITY, AMERICAN SOCIETY FOR MICROBIOLOGY. WASHINGTON, US, vol. 2, no. 68, February 2000 (2000-02), pages 688-693, XP002902371 ISSN: 0019-9567

## Description

### Field of the invention:

The present invention relates to a pharmaceutical composition useful for the treatment of invasive pulmonary aspergillosis in a subject.

Further, the present invention also relates to a method of treating invasive pulmonary aspergillosis in a subject.

More particularly, it relates to a method of treating invasive pulmonary aspergillosis in a subject using human mannan-binding lectin (MBL) protein.

### Background and prior art of the invention:

Mannan-binding lectin (MBL), an important constituent of innate immunity, belongs to the collectin family of proteins like the human lung surfactant proteins and bovine conglutinin.

Structurally, it is composed of trimeric subunits with a N-terminal collagenous section and a C-terminal globular carbohydrate-recognizing domain (CRD). MBL recognizes and binds to non-host carbohydrate structures expressed on the surfaces of various pathogens such as yeast, bacteria and viruses via its CRD and the collagenous domain reacts with the C 1 q receptor (collectin receptor) found on many cells including phagocytes, thus acting as an opsonin. It is known to enhance opsonophagocytosis (Jack et al, 2001) by binding to a wide range of clinically relevant organisms (Neth et al, 2000). After binding to its target, MBL initiates the lectin pathway of complement activation, which is independent of Clq and antibody and has been described as a key mechanism for the mammalian acute phase response to infection [Matsushuita et al, 1992]. This complement activation is mediated by distinct serine proteases [MBL-associated serine proteases, MASP-1 (Matsushita and Fujita, 1992], MASP-2 (Thiel et al, 1997), MASP-3 (Dahl et al, 2001) and Map 19 (Stover et al, 1999) a small protein of 19kDa. These serine proteases, upon binding to the microbial ligand, recruit the complement factors, C4 and C2, to generate the C3 convertase or directly activate C3 leading to either (a) phagocytosis of the opsonized target via the complent receptor, or (b) humoral cell killing via assembly of the membrane attack complex.

MBL is synthesized by hepatocytes and has been isolated from the liver or serum of several vertebrate species. Only one form of human MBL has been characterized, while two forms are found in rabbits, rats, mice and monkeys (Hansen et al, 1998). The MBL-A has been considered to be the serum form in rodents, whereas MBL-C has been earlier called the liver form (Oka et al, 1998), but it has been recently found that both forms appear in the serum (Hansen et al, 1990). The mouse serum concentrations of MBL-A are 7.5 ug/ml and MBL- C is 45 ug/ml (Liu et al, 2001), which is much higher than human serum MBL concentrations, which lie in the range of 0.3-4 ug/ml. This variation in human MBL serum levels is genetically determined. Three major mutations [codon 52 (CGT to TGT) (Madeson et al, 1994), codon 54 (GGC to GAC) (Sumiya et al, 1991) and codon 57 (GGA to GAA) (Lipscombe et al, 1992), resulting in Arg to Cys, Gly to Asp and Gly to Glu aminoacid substitutions], respectively have been found in exon 1 of the MBL gene, which encodes the collagenous domain of the protein (the collagen domain is important for the binding of MBL to the collectin receptors on different phagocytes, and for the activation of the complement). These structural polymorphisms along with two promoter polymorphisms [-550 (G to C), -221(G to C) and a SNP at +4 of the 5' untranslated region (C to T) (Madeson et al,) alter the structural assembly of MBL and lead to significantly reduced levels of the functional protein circulating in the serum. MBL gene heterogeneity and thus low levels of complement fixing MBL in the serum has been associated with a wide spectrum of bacterial and fungal diseases. Clinical studies have demonstrated a marked correlation between low MBL levels and immune opsonic deficiency (Super at al, 1989).

The natural human MBL protein is composed of up to 18 identical 32 kDa polypeptide chains (Lu et al, 1990), each comprising a short N-terminal segment of 21 amino acids including three cysteine residues, followed by 7 repeats of the collagenous motif Gly-X-Y interrupted by a Gln residues followed by another 12 Gly-X-Y repeats. The collagenous regions of the three- polypeptide chains combine to form a subunit, which is stabilised covalently by disulphide bridges. Individual subunits are joined by disulphide bridges as well as by non-covalent interactions (Lu et al, 1990). Clinical grade MBL has been obtained from blood donor plasma and shown to be safe upon infusion (Vaidimarsson et al, 1998). Production of recombinant MBL having a structure and an activity similar to that of native MBL has been attained successfully in human embryonic kidney cells followed by selective carbohydrate affinity chromatography (Vorup-Jensen at al, 2000). A European patent no: US2003191052 entitled: 'Novel indications of mannan-binding lectin (MBL) in the treatment of immunocompromised individuals' by SteffenThiel et al describes the potential use of human recombinant MBL (rMBL) in immunocompromised patients with low serum MBL levels to prevent these patients from bacterial and fungal infections. In this patent, the authors examine the influence of MBL deficiency (low MBL levels) on the occurrence of clinically significant infections in a group of hematologic individuals undergoing chemotherapy and show that the group with recurrent infections had significantly low levels of MBL. The description of the patent does not have any suitable example to verify their claims for therapeutic benefits of MBL in immunocompromised individuals with any microbial infection. To be precise, they did not show therapeutic benefits of MBL administration in immunocompromised patients with infections. The prior art US2003191052 in general claims all kinds of bacterial, fungal and viral infections with no supporting examples while our claims are very specific to invasive pulmonary aspergillosis treatment with MBL shown in an established murine model with comparable success to that of empirical antifungal drug Amphotericin B. Moreover, their study does not include patients with fungal infections. However, they have not actually used MBL for any treatment. Moreover, their study does not include patients with fungal infections. WO 03/090774 discloses the administration of pharmaceutical compositions comprising MASP- depleted MBL via parenteral intramuscular and intravenous routes for the treatment of infections from pathogens including Aspergillus fumigatus.

In recent years, bacterial and fungal infections have increased in alarming proportions and are the major cause of devastating illness, especially in the developing countries. Since the mid-1980's, fungal pathogens have begun to rival their bacterial counterparts. Species of the *Aspergillus* family account for a substantial number of these fungal infections and in particular *Aspergillus fumigatus* has emerged worldwide as a frequent cause of nosocomial infection in virtually every major medical center. The spectrum of disease caused by *Aspergillus* spp. is rather heterogeneous (Bardana et al, 1981) and depends upon the immunologic state of the patient. Lowered host resistance due to such factors as underlying debilitating disease, neutropenia chemotherapy, disruption of normal flora, and an inflammatory response due to the use of antimicrobial agents and steroids predisposes the patient to the invasive form of the disease whereas patients with pre-existing atopic tendencies may develop allergic and hypersensitivity reactions to inhaled conidia. *Aspergillus* spp. are increasingly recognized as major fungal pathogens in immunocompromised or neutropenic patients. As the incidence of AIDS, aplastic anemia, and organ transplantation increases and the use of chronic glucocorticoid treatment and aggressive antineoplastic chemotherapy regimes become more frequent, the number of patients susceptible to *Aspergillus* infection is rising. In immunocompromised or neutropenic patients, IPA, the most common form of the disease, is characterized by hyphal invasion and destruction of pulmonary tissue. Dissemination of *Aspergillus* infection to other organs occurs in approximately 20% of IPA cases (Bodey et al, 1989). In spite of correct diagnosis and treatment, IPA results in patient mortality of greater than 80%. The mortality rate among bone marrow transplantation patients can be as high as 95% (Sternberg, 1994). The crude mortality from invasive aspergillosis is around 85% when untreated and on treatment it falls to 50%. The treatment of choice in IPA is intravenous amphotericin B, which has been shown to reduce the mortality in patients of IPA. However, AmB is considered highly nephrotoxic, causing renal tubular acidosis, nephrocalcinosis, electrolyte disturbance, and decreased glomerular filtration (Maddux et al, 1980). The antifungal chemotherapy has often been found inadequate to completely eliminate infection in the immunocompromised subjects. Itraconazole has given good results in some cases but has yet to be fully evaluated in serious aspergillus infection.

Various studies have shown that the effector mechanisms of innate immunity mediated by phagocytes such as neutrophils and macrophages, pattern recognition molecules and complement constitutes an important line of defense against *Aspergillus fumigatus.* Therefore, there has been an intensive search for molecules that significantly enhance the contribution of the innate immune mechanisms against *Aspergillus* mediated infections. Previous studies have shown that one of the lung collectins, SP-D plays a significant defensive role against Aspergillus mediated infections (Madan et al, 2001). It has been demonstrated that the intranasal administration of recombinant SP-D in murine model of invasive pulmonary aspergillosis (IPA) leads to a survival rate of 80% in rSP-D treated IPA mice as compared to the untreated IPA mice. MBL, like SP-D has evolved as a key player of innate immunity and participates in a variety of host defense functions such as opsonization and complement activation, as has been mentioned earlier.

A series of in vitro interactions between *A. fumigatus* conidia and recombinant MBL have been studied. Both native and recombinant MBL significantly bound to the *A.fumigatus* conidia in a dose dependent manner in the presence of calcium and enhanced their agglutination. Further, MBL also enhanced complement dependent phagocytosis and killing of *A.fumigatus* conidia by PMNs, thus demonstrating a significant role of MBL in host defense against *A. fumigatus.* These in vitro studies demostrate that purified human MBL most effectively mediates the killing of the fungus in the range of 1-5 µg/ml and hence we selected two doses, that is 1 µg and 5 µg to establish the in vivo role of MBL in the murine model of invasive pulmonary aspergillosis (IPA).

However the prior art lacks in any report that specifies the protective role of MBL in the range of 1-5 µg/ 20 g of mice for rescuing 80 % of the mice suffering from invasive pulmonary aspergillosis similar to that observed in AmB-treated IPA mice. AmB is the comost common antifungal drug used these days. However, there are some serious side effects associated with continued use of AmB treatment such as nephrotoxicity, anemia, fever shaking chills, muscle or joint pain, headache, loss of appetite, nausea and vomiting. In the present invention, we describe for the first time the use of purified human recombinant MBL for treatment of invasive pulmonary aspergillosis in animal models. The utility of the present invention can be extended to human subjects suffering from invasive aspergillus infection.

### Objects of the invention:

The main object of the present invention is to provide a pharmaceutical composition useful for the treatment of invasive pulmonary aspergillosis.

Another object of the present invention is a method of treating invasive pulmonary aspergillosis in a subject.

Further another object of the present invention is to provide a method of treating invasive pulmonary aspergillosis in a subject using human mannan-binding lectin (MBL).

### Summary of the invention:

The present invention deals with a human mannose binding lectin protein for the treatment of invasive pulmonary aspergillosis in a subject, wherein the administration route is intranasally. The application deals with a pharmaceutical composition for the treatment of invasive pulmonary aspergillosis. It also provides a method of treating invasive pulmonary aspergillosis in a subject using human MBL protein. It may be useful for the treatment of individuals who are likely to contract such a condition due to treatment known to be associated with the occurrence of an immunosuppressed condition or otherwise. Examples of such treatments are e.g. chemotherapy and radiation therapy such as e.g. x-ray treatment. MBL is believed to exert its antimicrobial activity mainly through its opsonizing activity. This activity is dependent on activation of complement after binding of MBL to the microbial surface and deposition of C4b and C3b on the microorganism. MBL can also promote the direct complement-mediated killing of the microorganism through the activation of the terminal lytic pathway of complement and insertion of the membrane attack complex (MAC) in the membrane. It is possible according to the invention to therapeutically treat an invasive aspergillus infection by intranasal infusion of MBL.

### Brief description of the drawings:

Figure 1 represents the relative survival in different mice groups on different days. The survival rate is highest for 1 and 3 µg MBL (80%) treated IPA mice and amphotericin B treated IPA mice (80%) and lowest for the untreated IPA mice which showed 100% mortality on the 5^{th} day.
Figure 2 represents the comparative data of relative pulmonary fungal load given in terms of CFU counts per gram of the lung tissue on 4^{th} day after spore challenge. The CFU counts are highest for the untreated IPA mice, that is, 13.9 ± 0.7 and lowest for 3µg MBL treated IPA mice, that is, 4.2 ± 0.4.
Figure 3 demonstrates the fungal hyphae density as seen by the histopathological studies. The fungal density decreases in the MBL treated and amphotericin B treated IPA mice as compared to the untreated IPA mice.

### Detailed description of the invention:

Accordingly, the present invention provides a human mannose binding lectin protein for the treatment of invasive pulmonary aspergillosis in a subject, wherein the administration route is intranasally. The application provides a pharmaceutical composition useful for the treatment of invasive pulmonary aspergillosis in a subject wherein the said composition comprising the therapeutically effective amount of human mannose binding lectin protein or its derivatives or analogues or pharmaceutically acceptable salt thereof along with one or more pharmaceutically acceptable carriers.

In an embodiment of the present invention, the subject is human.

In another embodiment of the present invention, the dosage of the said composition is administered at a unit dose in the range of 1.0µg-5.0µg /20kg body weight.

Further, in another embodiment of the present invention, the said human MBL protein used is selected from the group consisting of native or recombinant, complete or a fragment of mannan binding lectin from any mammalian source.

Yet, in another embodiment of the present invention, the said carriers are selected from the group consisting of liposomes, microspheres and nanoparticles.

Still in another embodiment of the present invention, the human MBL protein is administered in the appropriate buffer soluble form wherein the buffer is selected from the group consisting of phosphate buffer saline (PBS), Tris buffer saline (TBS) of pH in the range of 7.0-8.0.

Still, in another embodiment of the present invention, the fungal hyphae of *Aspergillus fumigatus* are reduced in human MBL treated subject.

Still, in another embodiment of the present invention, the survival rate of human MBL treated subject is increased about 80%.

Still, in another embodiment of the present invention, 30- 35% pulmonary fungal load is reduced in human MBL treated subject.

Still, in another embodiment of the invention, the MBL protein administration reduces fungal hyphae to an extent similar to that of a known standard antifungal agent such as amphotericin B.

Further, the application also provides new use of human MBL protein in the treatment of invasive pulmonary aspergillosis in a subject.

In an embodiment of the present invention, the subject is human.

In another embodiment of the present invention, the said human MBL protein used is selected from the group consisting of native or recombinant, complete or a fragment of mannan binding lectin from any mammalian source.

For preparation of animal models of invasive pulmonary aspergillosis, BALB/c mice were divided into various experimental and control groups and subgroups as shown in Table I.

**Table I**

| **Mice Groups** | **No. of mice** | **Spore challenge intranasally in 50 µl saline on day 0** (**Day -1,0,1: immunosuppression with Wycort)** | **Intranasal administration of proteins/drugs in 50 µl saline/20kg body weight of mice on day 1** |
|---|---|---|---|
| Untreated IPA Control IPA | 10 6 | 1x 10⁸ spores saline | 50 µl of saline on day 1 |
| | | | 50 µl of saline on day 1 |
| AmB treated IPA AmB treated Control | 10 6 | 1x 10⁸ spores saline | 134.6 µg of AmB on day 1 |
| | | | 134.6 µg of AmB on day 1 |
| 1 □g rMBL treated IPA | 10 6 | 1x 10⁸ spores saline | 1µg of rMBL on day 1 |
| 1 □g rMBL treated Control | | | 1µg of rMBL on day 1 |
| 3 □g rMBL treated IPA | 10 6 | 1x10⁸ spores saline | 3 µg of rMBL on day 1 |
| 3 □g rMBL treated Control | | | 3 µg of rMBL on days 1 |
| 5 □g rMBL treated IPA | 10 | 1x10⁸ spores saline | 5 µg of rMBL on day 1 |
| 5 Og rMBL treated Control | 6 | | 5 µg of rMBL on day 1 |

### IPA: Invasive pulmonary aspergillosis, AmB: Amphotericin B, rMBL: recombinant MBL

The BALB/c mice were immunosuppressed by three intradermal injections of 2.5 mg of hydrocortisone acetate (Wycort) per 20 g mouse per day 1 day before, the day of, and the day after spore challenge.

On the day of spore challenge (day 0), mice were lightly anesthesized with ether and 10⁸ spores of *A. fumigatus* in 50 µl of sterile saline were administered intranasally in the groups of IPA mice, while 50 µl of saline alone was administered to the untreated control mice. The MBL-treated control and IPA mice received 1 µg of MBL in 50 µl of saline per mouse on day 1, and the groups of untreated control and untreated IPA mice received 50 µl of saline alone intranasally on day 1.

The MBL composition used for treatment can be produced from any MBL source available. The MBL source can be natural MBL, whereby the MBLs are produced in a native host organism, meaning that a cell normally expressing MBL produces MBL. One usual method of producing an MBL composition is by extraction of MBL from human body liquids, such as serum or plasma, but MBL may also be harvested from cultures of hepatocytes. In another aspect the MBL oligomers are produced by a host organism not natively expressing an MBL polypeptide, such as by recombinant technology, wherein the MBL source is the culture media from culturing of MBL producing cells. In the present experiments, the MBL used was in solution form produced by the recombinant technology.

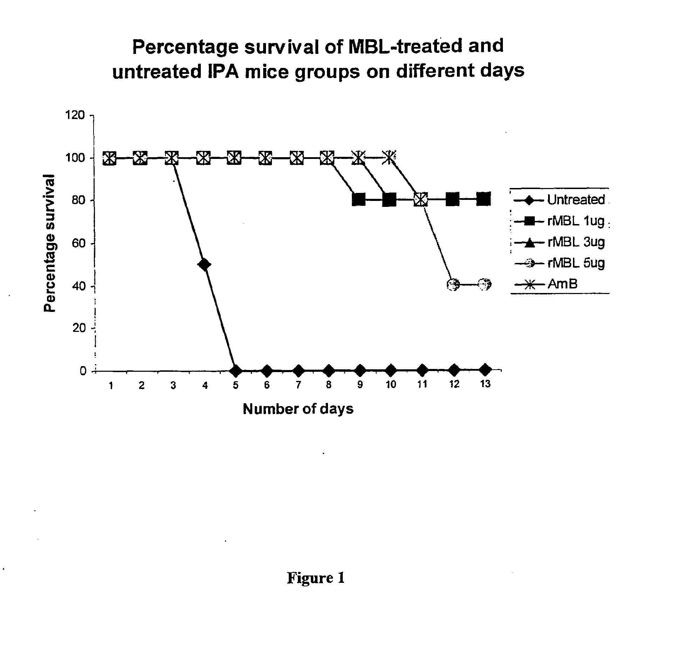

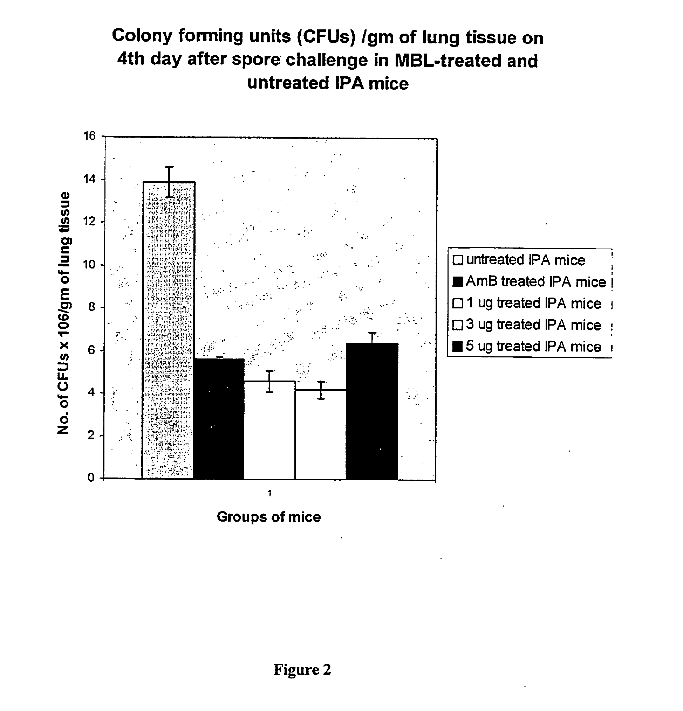

The survival rates of the MBL treated and untreated mice were monitored for 15 days (figure 1). Among the rest of the mice left in each group, two mice were sacrificed each on 4^{th} and 7^{th} day after fungal exposure and lung tissues were collected for histopathology and counting the colony forming units of the fungus (CFUs). For estimating the pulmonary fungal load and hyphae density of the mice, lungs were aseptically removed from each group of mice sacrificed on the 4^{th} and 7^{th} day, after the spore challenge. A portion of the lung tissue (10 mg) was homogenized with a mechanical homogenizer for 1 min in sterile PBS. The suspension was serially diluted 2-fold and incubated in triplicates on Sabouraud Agar (SDA) plates at 37°C. *A. fumigatus* colonies were counted after 24 hrs incubation and the values represent average ± standard deviation of six data points (three from each dilution). The number of Colony forming units (CFUs) that appeared on the plates corresponds to the pulmonary fungal load of different groups. The fungal load in terms of CFUs x 10⁶/gm of lung tissue in the untreated IPA mice, 13.9 ± 0.7 decreased to 4.6 ± 0.5 and 4.2 ± 0.4 in the 1 µg and 3 µg MBL treated IPA mice respectively and it was comparable to the amphotericin B treated IPA mice 5.65 ± 0.07 as shown in figure 2.

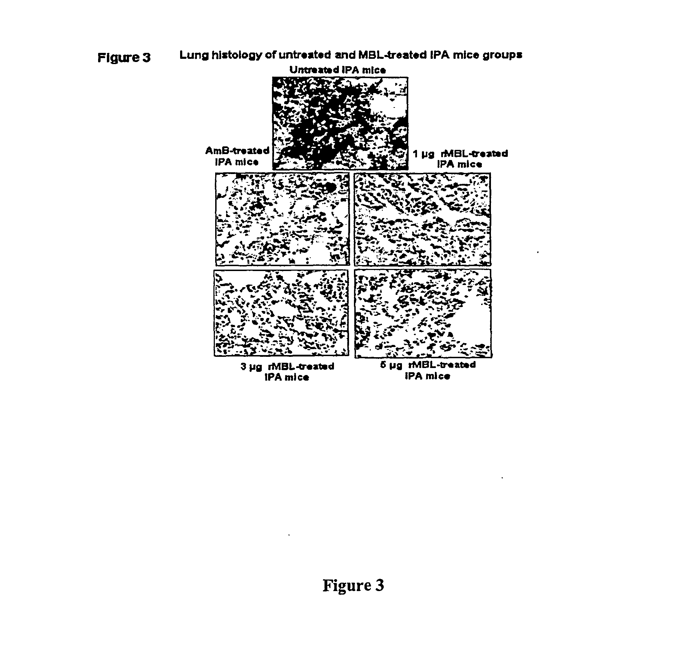

The other portion of the lung was kept in 10 % formalin for histopathological studies of the lung sections. There was a drastic decrease in the fungal hyphae density in the MBL treated and amphotericin B treated IPA mice as compared to the untreated mice (Figure 3).

The following examples are given by way of illustration of the present invention and should not be construed to limit the scope of present invention.

### EXAMPLE 1

### Preparation of animal model of invasive pulmonary aspergillosis

The animal model of invasive pulmonary aspergillosis is prepared by known methods of the immunosuppression of the mice (Allen et al, 1994) followed by exposure to *A. fumigatus* spores. The mice were immunosuppressed by three intradermal injections of 2.5 mg of hydrocortisone acetate (Wycort) per 20 g of mouse per day one day before, the day of, and the day after spore challenge (day-1,0, +1).

On the day of spore challenge (day 0), mice were lightly anesthesized with ether and 10⁸ spores of *A. fumigatus* in 50 µl of sterile saline were administered intranasally in the groups of IPA mice, while 50 µl of saline alone was administered to the untreated control mice. Table I describes the immunization protocol of different mice groups on different days.

### EXAMPLE 2

### Administration of rMBL

The administration of different concentrations of purified human rMBL (1-5 µg/mice) in saline intranasally to different groups of mice is described in Table I. The animal models of invasive pulmonary aspergillosis and their respective controls were given treatment with rMBL and amphotericin B. The amphotericin B treated mice acted as the positive control, since amphotericin B is a standard known antifungal agent.

The 1 µg rMBL-treated control and IPA mice received 1 µg of rMBL in 50 µl of saline per mouse on day 1. Similarly, 3 µg rMBL-treated control and IPA mice received 3 µg of rMBL in 50 µl of saline per mouse on day land 5 µg rMBL-treated control and IPA mice received 5 µg of rMBL in 50 µl of saline per mouse on day 1. The AmB-treated control and IPA mice received AmB (134.6 µg) only on day 1. The groups ofuntreated control and untreated IPA mice received 50 µl of saline without rMBL intranasally on day 1.

### EXAMPLE 3

### Estimating the fungal load

For estimating the pulmonary fungal load and hyphae density of the mice, lungs were aseptically removed from each group of mice sacrificed on the 4^{th} and 7^{th} day, after the spore challenge. A portion of the lung tissue (10 mg) was homogenized with a mechanical homogenizer for 1 min in sterile PBS. The suspension was serially diluted 2-fold and incubated in triplicate on Sabauraud Agar (SDA) plates at 37°C. *A. fumigatus* colonies were counted after 24 hrs incubation and the values represent average ± standard deviation of six data points (three from each dilution). The number of Colony forming units (CFUs) that appeared on the plates corresponds to the pulmonary fungal load of different groups.

### EXAMPLE 4

### Visualization of fungal hyphae

For visualization of the fungal hyphae in the lungs of the mice, a portion of the lungs collected from the mice sacrificed on 4^{th} and 7^{th} day from treated and untreated groups after spore challenge and trimmed of extraneous tissue was kept in 10 % formalin for histopathological studies and stored at 4°C. The tissue sections, made using a microtome and stained with hematoxylin and eosin, were examined at the magnification of 40X.

### EXAMPLE 5

### Calculation of the survival rate

Different groups of mice were monitored for 15 days for survival and thereby their survival rate was calculated.

### EXAMPLE 6

### Effect of MBL treatment

The BALB/c mice were divided into different groups as given in Table I and immunosuppressed by three intradermal injections of 2.5 mg of hydrocortisone acetate (Wycort) per 20 kg mouse per day 1 day before, the day of, and the day after spore challenge. On the day of spore challenge (day 0), mice were lightly anesthesized with ether and 10⁸ spores of *A. fumigatus* in 50 µl of sterile PBS were administered intranasally in the groups of IPA mice, while 50 µl of PBS was administered to the control mice. The invasive pulmonary aspergillosis affected animal model is a model wherein the fungal hyphae are visible in the lung tissue upon histopathological examination and the survival of the affected animal is not more than 5-7 days. After the preparation of the animal models, different concentrations of purified human rMBL (1-5 µg/mice) were prepared in solution form in phospahate buffer saline (PBS), pH 7.2. The MBL-treated control and IPA mice received 1 µg of MBL in 50 µl of PBS per mouse on day 1, and the groups of untreated control and untreated IPA mice received 50 µl of PBS alone intranasally on day 1. Similarly, 3 µg rMBL-treated control and IPA mice received 3 µg of rMBL in 50 µl of PBS per mouse on day 1 and 5 µg rMBL-treated control and IPA mice received 5 µg of rMBL in 50 µl of PBS per mouse on day 1. The AmB-treated control and IPA mice received AmB (134.6 µg) only on day 1. The groups of untreated control and untreated IPA mice received 50 µl of PBS alone intranasally on day 1. After treatment with MBL, the pulmonary fungal load was estimated and fungal hyphae were visualized in the lung tissue of MBL-treated and untreated mice. Survival rate of the MBL treated mice was compared with that of the untreated mice.

There was an increase in the percentage of survival in the MBL treated IPA mice. All of the control mice, which received no spores and were treated with PBS, AmB or rMBL, showed 100% survival. The untreated IPA mice showed 100% mortality by the 5th day. Survival in the AmB-treated IPA mice 1µg and the 3 µg rMBL-treated groups of IPA mice was the same (80% survival) and was significantly different from that of untreated IPA mice (P < 0.025). Survival in the 5 µg of rMBL treated group of IPA mice (40%) was higher than in the untreated group of IPA mice (*P* < 0.500) but was reduced compared to survival in the AmB and 1 µg of rMBL-treated groups of IPA mice.

There was a reduction in the pulmonary fungal load expressed in terms of CFU counts/gm of lung tissue in the MBL treated IPA mice as compared to the untreated IPA mice.

There was a decrease in the fungal hyphae density in the lungs of MBL treated IPA mice as compared to the untreated IPA mice as seen in the histopathological studies.

### EXAMPLE 7

### Effect of MBL treatment

The BALB/c mice were divided into different groups as given in Table I and immunosuppressed by three intradermal injections of 2.5 mg of hydrocortisone acetate (Wycort) per 20 kg mouse per day 1 day before, the day of, and the day after spore challenge. On the day of spore challenge (day 0), mice were lightly anesthesized with ether and 10⁸ spores of *A. fumigatus* in 50 µl of sterile PBS were administered intranasally in the groups of IPA mice, while 50 µl of PBS was administered to the control mice. The invasive pulmonary aspergillosis affected animal model is a model wherein the fungal hyphae are visible in the lung tissue upon histopathological examination and the survival of the affected animal is not more than 5-7 days. After the preparation of the animal models, different concentrations of purified human rMBL (1-5 µg/mice) were prepared in solution form in phospahate buffer saline (PBS), pH: 7.4. The MBL-treated control and IPA mice received 1 µg of MBL in 50 µl of PBS per mouse on day 1, and the groups of untreated control and untreated IPA mice received 50 µl of PBS alone intranasally on day 1. Similarly, 3 µg rMBL-treated control and IPA mice received 3 µg of rMBL in 50 µl of PBS per mouse on day 1 and 5 µg rMBL-treated control and IPA mice received 5 µg of rMBL in 50 µl of PBS per mouse on day 1. The AmB-treated control and IPA mice received AmB (134.6 µg) only on day 1. The groups ofuntreated control and untreated IPA mice received 50 µl of PBS alone intranasally on day 1. After treatment with MBL, the pulmonary fungal load was estimated and fungal hyphae were visualized in the lung tissue of MBL-treated and untreated mice. Survival rate of the MBL treated mice was compared with that of the untreated mice.

There was an increase in the percentage of survival in the MBL treated IPA mice as shown in Figure 1. All of the control mice, which received no spores and were treated with PBS, AmB or rMBL, showed 100% survival. The untreated IPA mice showed 100% mortality by the 5th day. Survival in the AmB-treated IPA mice and the 1 µg rMBL-treated groups of IPA mice was the same (80% survival) and was significantly different from that of untreated IPA mice (P < 0.025). Survival in the 5 µg of rMBLtreated group of IPA mice (40%) was higher than in the untreated group of IPA mice (*P* < 0.500) but was reduced compared to survival in the AmB and 1 µg of rMBL-treated groups of IPA mice.

There was a reduction in the pulmonary fungal load expressed in terms of CFU counts/gm of lung tissue inthe MBL treated IPA mice as compared to the untreated IPA mice. The untreated IPA mice showed high levels of CFUs (CFUs x 10⁶ ) in the lung homogenates (13.9 ± 0.7) x 106/gm of lung tissue) than the AmB treated (5.65 ± 0.07) ,1 µg rMBLtreated (4.6 ± 0.5), 3 µg rMBL (4.2 ± 0.4) and 5 µg rMBL (6.4 ± 0.5) treated IPA mice. Figure 2 shows the relative CFU counts x 10⁶ /gm of lung tissue in different groups of treated and untreated IPA mice on 4^{th} day after spore challenge.

There was a decrease in the fungal hyphae density in the lungs of MBL treated IPA mice as compared to the untreated IPA mice as seen in the histopathological studies shown in Figure 3.

### EXAMPLE 8

### Effect of MBL treatment

The BALB/c mice were divided into different groups as given in Table I and immunosuppressed by three intradermal injections of 2.5 mg of hydrocortisone acetate (Wycort) per 20 g mouse per day 1 day before, the day of, and the day after spore challenge. On the day of spore challenge (day 0), mice were lightly anesthesized with ether and 10⁸ spores of *A. fumigatus* in 50 µl of sterile TBS were administered intranasally in the groups of IPA mice, while 50 µl of TBS was administered to the control mice. The invasive pulmonary aspergillosis affected animal model is a model wherein the fungal hyphae are visible in the lung tissue upon histopathological examination and the survival of the affected animal is not more than 5-7 days. After the preparation of the animal models, different concentrations of purified human rMBL (1-5 µg/mice) were prepared in solution form in Tris buffer saline (TBS), pH: 7.6.

The MBL-treated control and IPA mice received 1 µg of MBL in 50 µl of TBS per mouse on day 1, and the groups of untreated control and untreated IPA mice received 50 µl of TBS alone intranasally on day 1. Similarly, 3 µg rMBL-treated control and IPA mice received 3 µg of rMBL in 50 µl of TBS per mouse on day 1 and 5 µg rMBL-treated control and IPA mice received 5 µg of rMBL in 50 µl of TBS per mouse on day 1. The AmB-treated control and IPA mice received AmB (134.6 µg) only on day 1. The groups of untreated control and untreated IPA mice received 50 µl of TBS alone intranasally on day 1. After treatment with MBL, the pulmonary fungal load was estimated and fungal hyphae were visualized in the lung tissue of MBL-treated and untreated mice. Survival rate of the MBL treated mice was compared with that of the untreated mice.

There was an increase in the percentage of survival in the MBL treated IPA mice. All of the control mice, which received no spores and were treated with TBS, AmB or rMBL, showed 100% survival. The untreated IPA mice showed 100% mortality by the 5th day. Survival in the AmB-treated IPA mice and the 1 µg rMBL-treated groups of IPA mice was the same (80% survival) and was significantly different from that of untreated IPA mice (P < 0.025). Survival in the 5 µg of rMBLtreated group of IPA mice (40%) was higher than in the untreated group of IPA mice (*P* < 0.500) but was reduced compared to survival in the AmB and 1 µg of rMBL-treated groups of IPA mice.

There was a reduction in the pulmonary fungal load expressed in terms of CFU counts/gm of lung tissue inthe MBL treated IPA mice as compared to the untreated IPA mice.

There was a decrease in the fungal hyphae density in the lungs of MBL treated IPA mice as compared to the untreated IPA mice as seen in the histopathological studies.

### EXAMPLE 9

### Effect of MBL treatment

The BALB/c mice were divided into different groups as given in Table I and immunosuppressed by three intradermal injections of 2.5 mg of hydrocortisone acetate (Wycort) per 20 g mouse per day 1 day before, the day of, and the day after spore challenge. On the day of spore challenge (day 0), mice were lightly anesthesized with ether and 10⁸ spores of *A. fumigatus* in 50 µl of sterile TBS were administered intranasally in the groups of IPA mice, while 50 µl of TBS was administered to the control mice. The invasive pulmonary aspergillosis affected animal model is a model wherein the fungal hyphae are visible in the lung tissue upon histopathological examination and the survival of the affected animal is not more than 5-7 days. After the preparation of the animal models, different concentrations of purified human rMBL (1-5 ug/mice) were prepared in solution form in Tris buffer saline (TBS), pH: 8.0.

The MBL-treated control and IPA mice received 1 µg of MBL in 50 µl of TBS per mouse on day 1, and the groups of untreated control and untreated IPA mice received 50 µl of TBS alone intranasally on day 1. Similarly, 3 µg rMBL-treated control and IPA mice received 3 µg of rMBL in 50 µl of TBS per mouse on day 1 and 5 µg rMBL-treated control and IPA mice received 5 µg of rMBL in 50 µl of TBS per mouse on day 1. The AmB-treated control and IPA mice received AmB (134.6 µg) only on day 1. The groups of untreated control and untreated IPA mice received 50 µl of TBS alone intranasally on day 1. After treatment with MBL, the pulmonary fungal load was estimated and fungal hyphae were visualized in the lung tissue of MBL-treated and untreated mice. Survival rate of the MBL treated mice was compared with that of the untreated mice.

There was an increase in the percentage of survival in the MBL treated IPA mice. All of the control mice, which received no spores and were treated with TBS, AmB or rMBL, showed 100% survival. The untreated IPA mice showed 100% mortality by the 5th day. Survival in the AmB-treated IPA mice and the 1µg rMBL-treated groups of IPA mice was the same (80% survival) and was significantly different from that of untreated IPA mice (*P* < 0.025). Survival in the 5 µg of rMBLtreated group of IPA mice (40%) was higher than in the untreated group of IPA mice (*P* < 0.500) but was reduced compared to survival in the AmB and 1 µg of rMBL-treated groups of IPA mice.

There was a reduction in the pulmonary fungal load expressed in terms of CFU counts/gm of lung tissue inthe MBL treated IPA mice as compared to the untreated IPA mice.

There was a decrease in the fungal hyphae density in the lungs of MBL treated IPA mice as compared to the untreated IPA mice as seen in the histopathological studies.

### Advantages:

The main advantages of the present invention are:
- The present invention provides the pharmaceutical composition useful for the treatment of invasive pulmonary aspergillosis, which is administered intranasally.

## Claims

1. A human mannose binding lectin protein for the treatment of invasive pulmonary aspergillosis in a subject, wherein the administration route is intranasally.

2. The human mannose binding lectin protein for the treatment as claimed in claim 1, wherein the subject is human.

3. The human mannose binding lectin protein for the treatment as claimed in claims 1 or 2, wherein the human MBL protein is administered in the appropriate buffer soluble form, wherein the buffer is selected from the group consisting of phosphate buffer saline (PBS), Tris buffer saline (TBS) of pH in the range of 7.0-8.0.

## Patentansprüche

1. Humanes Mannose-bindendes Lektinprotein zur Behandlung einer atemwegsinvasiven Aspergillose in einem Patienten, wobei die Verabreichungsform intranasal ist.

2. Das humane Mannose-bindende Lektinprotein zur Behandlung wie in Anspruch 1 beansprucht, wobei der Patient ein Mensch ist.

3. Das humane Mannose-bindende Lektinprotein zur Behandlung wie in Anspruch 1 oder 2 beansprucht, wobei das humane MBL Protein in der geeigneten pufferlöslichen Form verabreicht wird, wobei der Puffer ausgewählt ist aus der Gruppe bestehend aus phosphatgepufferter Salzlösung (PBS), Tris-gepufferter Salzlösung (TBS) mit einem pH im Bereich von 7.0-8.0.

## Revendications

1. Protéine humaine lectine de liaison du mannose pour le traitement de l'aspergillose pulmonaire invasive chez un sujet, où la vole d'administration est intranasale.

2. Protéine humaine lectine de liaison du mannose pour le traitement selon la revendication 1, où le sujet est un être humain.

3. Protéine humage lectine de liaison du mannose pour le traitement selon la revendication 1 ou 2, où la protéine humaine lectine de liaison du mannose MBL est administrée sous la forme soluble dans un tampon approprié, où le tampon est sélectionné dans le groupe composé de la solution saline de tampon phosphate (PBS), de la solution saline de tampon Tris (TBS) de pH dans l'intervalle de 7,0-8,0.
